# EUROPÄISCHE PATENTSCHRIFT

(11) **EP 0 570 375 B1**
(45) Veröffentlichungstag und Bekanntmachung des Hinweises auf die Patenterteilung: **19.04.1995**
(21) Anmeldenummer: 91920137.6
(22) Anmeldetag: 21.11.1991
(51) Int. Cl.: C07K 7/06, A61K 38/55

(54) **NEUE ANTIKOAGULATORISCH WIRKSAME PEPTIDE**
NOVEL PEPTIDES WITH ANTICOAGULATORY EFFECT
NOUVEAUX PEPTIDES A ACTIVITE ANTICOAGULANTE

(30) Priorität: 07.02.1991 DE 4103649
(43) Veröffentlichungstag der Anmeldung: 24.11.1993
(73) Patentinhaber: BASF Aktiengesellschaft, 67063 Ludwigshafen (DE)
(72) Erfinder: SCHMIED, Bernhard, D-6710 Frankenthal (DE); HOEFFKEN, Hans, Wolfgang, D-6700 Ludwigshafen (DE); HORNBERGER, Wilfried, D-6700 Ludwigshafen (DE); BERNARD, Harald, D-6702 Bad Duerkheim (DE)
(86) Internationale Anmeldenummer: EP9102191
(87) Internationale Veröffentlichungsnummer: WO9213879

(56) Entgegenhaltungen:
- EP-A- 372 670
- EP-A- 443 429
- EP-A- 443 598
- WO-A-90/03391
- WO-A-91/01328

## Beschreibung

Hirudin ist ein Naturstoff mit blutgerinnungshemmenden Eigenschaften, der in den Speicheldrüsen des Blutegels Hirudo medicinalis gebildet wird. Hirudin liegt als Gemisch aus Polypeptiden mit 64 bis 66 Aminosäuren vor. Hauptbestandteil dieses Gemisches ist ein Polypeptid mit 65 Aminosäuren, das an Tyrosin-63 einen O-Sulfatrest trägt:

Val-Val-Tyr-Thr-Asp-Cys-Thr-Glu-Ser-Gly-
Gln-Asn-Leu-Cys-Leu-Cys-Glu-Gly-Ser-Asn-
Val-Cys-Gly-Gln-Gly-Asn-Lys-Cys-Ile-Leu-
Gly-Ser-Asp-Gly-Glu-Lys-Asn-Gly-Cys-Val-
Thr-Gly-Glu-Gly-Thr-Pro-Lys-Pro-Gln-Ser-
His-Asn-Asp-Gly-Asp-Phe-Glu-Glu-Ile-Pro-
Glu-Glu-Tyr(SO3H)-Leu-Gln

Hirudin bindet sehr stark und mit hoher Spezifität an Thrombin, das Schlüsselenzym der Gerinnungskaskade. Der Thrombin-Hirudin-Komplex ist nicht sehr zur Spaltung von fibrinogen, dem natürlichen Substrat von Thrombin, befähigt.

Die antikoagulatorische Wirkung läßt Hirudin als potentielles Pharmakon erscheinen. Allerdings kann natürliches (O-sulfatiertes) Hirudin nur sehr mühsam und in sehr geringen Ausbeuten aus Blutegeln isoliert werden. Rekombinantes Hirudin enthält keinen sulfatierten Tyrosinrest und besitzt eine deutlich schlechtere Bindungskonstante gegenüber Thrombin.

Hirudin, das für medizinische Anwendungen bestimmt ist und das aus zellulärem Material isoliert wurde, muß mit besonderer Sorgfalt gereinigt werden. Rest-DNA, Fremdproteine, Toxine oder virale Kontaminationen sind unter allen Umständen auszuschließen. Darüberhinaus ist eine reproduzierbare perorale oder transdermale Applikation für ein Polypeptid dieser Größe, selbst unter Anwendung galenischer Hilfsmittel, kaum vorstellbar.

Aus den genannten Gründen wurde schon frühzeitig versucht aus der Hirudin-Sequenz Oligopeptide abzuleiten, die ebenfalls über antikoagulatorische Eigenschaften verfügen, aber vollsynthetisch und damit preisgünstig und in hoher Reinheit herzustellen sind.

Viele dieser literaturbekannten Peptide weisen tatsächlich thrombininhibierende Eigenschaften auf (J.Med.Chem. 31, 1009 (1988); EP 291981). Allerdings fällt die Wirkstärke dieser Verbindungen mit abnehmender Kettenlänge stark ab. Die EC₁₀₀ des C-terminalen Dekapeptids Hirudin₅₆₋₆₅ liegt etwa 2000mal höher als die des Hirudins.

Es wurde nun gefunden, daß bei geeigneter Substitution die thrombininhibierende Wirkung kurzer Peptide, die sich vom C-Terminus des Hirudin ableiten, dieselbe Größenordnung der Wirkstärke wie Hirudin erreichen können.

Gegenstand der Erfindung sind Peptide der Formel I 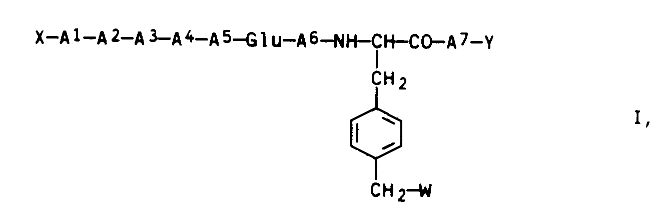
worin
- X: H, Acetyl, Succinyl, Maleoyl, Phthaloyl oder Fumaryl,
- A¹: Phe, Tyr oder Cha,
- A²: Glu oder D-Glu,
- A³: Glu, Pro oder Hyp,
- A⁴: Ile, Leu, Val oder Cha,
- A⁵: Pro oder Hyp,
- A⁶: Glu oder D-Glu,
- A⁷: Leu, D-Leu, Ile, D-Ile, Cha oder D-Cha,
- Y: OH, Gln-OH, Gln-NH₂, D-Gln-OH, D-Gln-NH₂, Glu-OH, Glu-NH₂, D-Glu-OH oder D-Glu-NH₂ und
- W: für einen der Reste SO₃H oder PO₃H₂ stehen,
sowie deren Salze mit physiologisch verträglichen Basen oder Säuren.

Zur Abkürzung der Aminosäuren wurde der übliche Dreibuchstaben-Code benutzt, Cha ist Cyclohexylalanin und Hyp ist trans-4-Hydroxyprolin. Falls nicht anders spezifiziert, liegen die Aminosäuren in der L-Konfiguration vor. Die vom Phenylalanin abgeleiteten unnatürlichen Aminosäuren mit den Resten W können sowohl in der L- als auch in der D-Konfiguration vorliegen.

In der Formel I bedeuten X vorzugsweise Succinyl, A¹ vorzugsweise Tyr, A² vorzugsweise Glu, A³ vorzugsweise Pro oder Hyp, A⁴ vorzugsweise Ile, Val oder Cha, A⁵ vorzugsweise Hyp, A⁶ vorzugsweise Glu, A⁷ vorzugsweise Leu, Ile oder Cha und Y vorzugsweise OH, Gln-OH, D-Gln-OH, Glu-OH oder D-Glu-OH.

Als physiologisch verträgliche Säuren sind insbesondere zu nennen: Salzsäure, Zitronensäure, Weinsäure, Milchsäure, Phosphorsäure, Methansulfonsäure, Essigsäure, Ameisensäure, Maleinsäure, Fumarsäure, Apfelsäure, Bernsteinsäure, Malonsäure, Schwefelsäure, L-Glutaminsäure, L-Asparaginsäure, Brenztraubensäure, Schleimsäure, Benzoesäure, Glucuronsäure, Oxalsäure, Ascorbinsäure, Acetylglycin.

Als physiologisch verträgliche Basen kommen Hydroxide und Hydrogencarbonate folgender Substanzen in Betracht: Aluminium, Calcium, Kalium, Lithium, Magnesium, Natrium, Diethanolamin, Ethylendiamin und Meglumin.

Die neuen Verbindungen lassen sich nach in der Peptidchemie bekannten Methoden herstellen.

So kann man die Peptide sequentiell aus Aminosäuren oder durch Fragmentverknüpfung geeigneter Peptide aufbauen. Beim sequentiellen Aufbau wird die Peptidkette beginnend am C-Terminus stufenweise um jeweils eine Aminosäure verlängert. Bei der Fragmentkupplung können Fragmente unterschiedlicher Länge miteinander verknüpft werden, wobei die Fragmente wiederum durch sequentiellen Aufbau aus Aminosäuren oder ihrerseits durch Fragmentkupplung gewonnen werden können.

Sowohl beim sequentiellen Aufbau, als auch bei der Fragmentkupplung müssen die Bausteine durch Bildung einer Amidbindung verknüpft werden. Hierzu eignen sich enzymatische und chemische Methoden.

Chemische Methoden zur Amidbindungsbildung sind ausführlich behandelt bei Müller, Methoden der Organischen Chemie Vol XV/2, pp 1-364, Thieme Verlag, Stuttgart, 1974; Stewart, Young, Solid Phase Peptide Synthesis, pp 31-34, 71-82, Pierce Chemical Company, Rockford, 1984; Bodanszky, Klausner, Ondetti, Peptide Synthesis, pp 85-128, John Wiley & Sons, New York, 1976 und anderen Standardwerken der Peptidchemie. Besonders bevorzugt sind die Azidmethode, die symmetrische und gemischte Anhydridmethode, in situ erzeugte oder präformierte Aktivester und die Amidbindungsbildung mit Hilfe von Kupplungsreagenzien (Aktivatoren), insbesondere Dicyclohexylcarbodiimid (DCC), Diisopropylcarbodiimid (DIC), 1-Ethoxycarbonyl-2-ethoxy-1,2-dihydrochinolin (EEDQ), 1-Ethyl-3-(3-dimethylaminopropyl)-carbodiimidhydrochlorid (EDCI), n-Propanphosphonsäureanhydrid (PPA), N,N-Bis(2-oxo-3-oxazolidinyl)amidophosphorsäurechlorid (BOP-Cl), Diphenylphosphorylazid (DPPA), Castro's Reagenz (BOP), 2(1H-Benzotriazol-(1)-yl)-1,1,3,3-tetramethyluronium-Salze (HBTU), 2,5-Diphenyl-2,3-dihydro-3-oxo-4-hydroxythiophendioxid (Steglichs Reagenz; HOTDO) und 1,1'-Carbonyl-diimidazol (CDI). Die Kupplungsreagenzien können allein oder in Kombination mit Additiven wie N,N'-Dimethyl-4-aminopyridin (DMAP), N-Hydroxybenzotriazol (HOBt), N-Hydroxybenzotriazin (HOOBt), N-Hydroxysuccinimid (HOSu) oder 2-Hydroxypyridin eingesetzt werden.

Während bei der enzymatischen Peptidsynthese normalerweise auf Schutzgruppen verzichtet werden kann, ist für die chemische Synthese ein reversibler Schutz der an der Bildung der Amidbindung nicht beteiligten reaktiven funktionellen Gruppen der beiden Reaktionspartner erforderlich. Bei den anspruchsgemäßen Verbindungen der Formel I kommt dem derivatisierten N-Terminus zusätzlich eine, die Wirkstärke modulierende, Bedeutung zu.

Bei den chemischen Peptidsynthesen werden drei literaturbekannte Schutzgruppentechniken bevorzugt: Die Benzyloxycarbonyl(Z)-, die t-Butyloxycarbonyl(Boc)- und die 9-Fluorenylmethyloxycarbonyl(Fmoc)-Schutzgruppentechnik. Bezeichnet ist jeweils die Schutzgruppe der α-Aminofunktion des kettenverlängernden Bausteines. Die Seitenkettenschutzgruppen der trifunktionellen Aminosäuren werden so gewählt, daß sie nicht notwendigerweise zusammen mit der α-Aminoschutzgruppe abgespalten werden. Eine ausführliche Übersicht über Aminosäureschutzgruppen geben Müller, Methoden der Organischen Chemie Vol XV/1, pp 20-906, Thieme Verlag, Stuttgart, 1974 und Bodanszky, The Practice of Peptide Synthesis, Springer-Verlag, Berlin 1984. Die Bausteine, die dem Aufbau der Peptidkette dienen, können in Lösung, in Suspension oder nach einem ähnlichen Verfahren, wie es von Merrifield in J. Amer. Chem. Soc. 85, 2149, 1963 beschrieben ist, zur Reaktion gebracht werden. Besonders bevorzugt sind Verfahren, bei denen Peptide sequentiell oder durch Fragmentkupplung unter Verwendung der Z-, Boc- oder Fmoc-Schutzgruppentechnik aufgebaut werden, wobei die Reaktionspartner in Lösung zur Reaktion gebracht werden, sowie Verfahren, bei denen, ähnlich der genannten Merrifield-Technik, ein Reaktionspartner an einen unlöslichen polymeren Träger (im folgenden auch Harz genannt) gebunden zur Reaktion gebracht wird. Dabei wird das Peptid typischerweise unter Verwendung der Boc- oder Fmoc-Schutzgruppentechnik sequentiell am polymeren Träger aufgebaut, wobei die wachsende Peptidkette am C-Terminus kovalent mit den unlöslichen Harzteilchen verbunden ist. Diese Arbeitsweise erlaubt es, Reagentien und Nebenprodukte durch Filtration zu entfernen, die Umkristallisation von Zwischenprodukten wird somit überflüssig.

Die geschützten Aminosäuren können an beliebige geeignete Polymerisate gebunden werden, die lediglich in den verwendeten Lösungsmitteln unlöslich sein und eine beständige physikalische Form, die leichte Filtration ermöglicht, aufweisen müssen. Das Polymerisat muß eine funktionelle Gruppe enthalten, an die die erste geschützte Aminosäure durch eine kovalente Bindung fest gebunden werden kann. Für diesen Zweck eignen sich die verschiedensten Polymerisate, z.B. Cellulose, Polyvinylalkohol, Polymethacrylat, sulfoniertes Polystyrol, chlormethyliertes Copolymerisat von Styrol und Divinylbenzol (Merrifield-Harz), 4-Methylbenzhydrylamin-Harz (MBHA-Harz), Phenylacetamidomethyl-Harz (Pam-Harz), p-Benzyloxybenzylalkohol-Harz, Benzhydrylamin-Harz (BHA-Harz), 4-(Hydroxymethyl)-benzoyloxymethyl-Harz, Harz nach Breipohl et al. (Tetrahedron Lett. 28, 565, 1987; Fa. BACHEM), HYCRAM-Harz (Fa. ORPEGEN) oder SASRIN-Harz (Fa. BACHEM).

Für die Peptidsynthese in Lösung eignen sich alle Lösungsmittel, die sich unter den Reaktionsbedingungen als inert erweisen, insbesondere Wasser, N,N'-Dimethylformamid (DMF), Dimethylsulfoxid (DMSO), Acetonitril, Dichlormethan (DCM), 1,4-Dioxan, Tetrahydrofuran (THF), N-Methyl-2-pyrrolidon (NMP) sowie Gemische der genannten Lösungsmittel. Die Peptidsynthese am polymeren Träger kann in allen inerten organischen Lösungsmitteln, in denen die verwendeten Aminosäurederivate löslich sind, durchgeführt werden; bevorzugt sind jedoch Lösungsmittel, die zusätzlich harzquellende Eigenschaften besitzen, wie DMF, DCM, NMP, Acetonitril und DMSO, sowie Gemische dieser Lösungsmittel.

Nach erfolgreicher Synthese wird das Peptid vom polymeren Träger abgespalten. Die Bedingungen, unter denen sich die verschiedenen Harztypen abspalten lassen, sind literaturbekannt. Am häufigsten finden saure und Palladium-katalysierte Spaltreaktionen Anwendung, insbesondere die Spaltung in flüssigem wasserfreiem Fluorwasserstoff, in wasserfreier Trifluormethansulfonsäure, in verdünnter oder konzentrierter Trifluoressigsäure oder die Palladium-katalysierte Spaltung in THF oder THF-DCM-Gemischen in Anwesenheit einer schwachen Base wie z.B. Morpholin. Je nach Wahl der Schutzgruppen können diese unter den Spaltbedingungen erhalten bleiben oder ebenfalls abgespalten werden. Auch eine teilweise Entschützung des Peptids kann sinnvoll sein, oder die postsynthetische Derivatisierung des bei der Spaltung freigesetzten N-Terminus, wenn die Eigenschaften der Verbindungen dadurch günstig beeinflußt werden.

Alle für die Peptidsynthese verwendeten Bausteine sind kommerziell verfügbar, mit Ausnahme der Aminosäuren der Formel II (worin W die in Formel I angegebene Bedeutung besitzt) 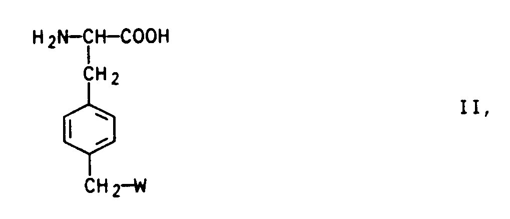
die über die Acetamidomalonester-Synthese oder andere literaturbekannte Methoden (E. Müller, Methoden der organischen Chemie, Bd XI/2; 1958) zur Darstellung von Aminosäuren zugänglich sind.

In Abhängigkeit von den Edukten und den Reaktionsbedingungen fallen die Aminosäuren der Formel II entweder mit definierter Stereochemie oder racemisch an. Die racemischen Aminosäuren der Formel II lassen sich nach literaturbekannten Methoden in ihre Antipoden trennen oder die Racemate werden in die Peptidsynthese eingesetzt und die resultierenden diastereomeren Peptidgemische getrennt.

Die biologische Charakterisierung der neuen Peptide als Thrombin-Inhibitoren erfolgte in folgenden Testsystemen:

### 1. Thrombinzeit (TT) in vitro

Zur Gewinnung von Citratplasma wird humanes Blut durch Venenpunktion am Arm entnommen, mit Natriumcitrat (0,11 mol/l) gemischt (9 Teile Blut + 1 Teil Natriumcitrat) und anschließend 10 min bei 1600 xg bei Raumtemperatur zentrifugiert. Zu 50 »l der Substanzlösung bzw. Lösungsmittel werden 50 »l Citratplasma gegeben und 2 min bei 37°C inkubiert. Danach werden 100 »l auf 37°C temperiertes Thrombin-Reagenz (Boehringer Mannheim) zupipettiert und die Zeit bis zum Eintritt der Gerinnung in einem photometrischen Koagulatometer gemessen.

Als Maß der relativen Wirksamkeit wird als EC₁₀₀ die Konzentration einer Prüfsubstanz in mol/l berechnet, die die Plasma-Thrombinzeit um 100 % verlängert.

Die Plasma-Thrombinzeit erfaßt die Thrombin-induzierte Fibrinbildung aus Fibrinogen und die Fibrinaggregation, d.h. den letzten Schritt der Gerinnung.

### 2. Amidolytische Bestimmung der Aktivität von Thrombin (analog auch von anderen Serinproteasen: Trypsin, Chymotrypsin, Plasmin, Faktor Xa, aktives Protein C)

Testprinzip:

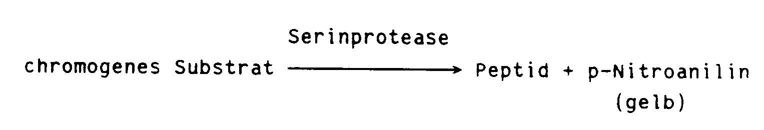

In Mikroplatten werden jeweils 250 »l Thrombin (0,124 IU/ml, EK 0,1 IU/ml) in Tris-Puffer (Tris 50 mmol/l, NaCl 154 mmol/l, pH 8,0) vorgelegt. Diese Lösung wird mit 10 »l Lösungsmittel (Kontrolle) bzw. Prüfsubstanz versetzt, 1 min gemischt und 4 min bei 25°C inkubiert. Danach wird die Reaktion durch Zugabe von 50 »l Substratlösung (S-2238, 0,62 mmol/l, EK 0,1 mmol/l) gestartet und nach kurzem Mischen bei 25°C inkubiert. Nach 5 min wird die Reaktion durch Zugabe von 50 »l 35 %iger Essigsäure gestoppt und die Extinktion bei 405 nm (gegen 630 nm) gemessen. Die nach Reaktionsende gemessene Extinktion ist proportional der Enzymaktivität.

Als Maß der relativen Wirksamkeit wird als IC₅₀ die Konzentration einer Prüfsubstanz in mol/l berechnet, die die Enzymaktivität um 50 % vermindert.

Analog zu Thrombin wird auch die Wirkung auf die amidolytische Aktivität von anderen Serinproteasen

| | |
|---|---|
| Trypsin (0,1 mg/l) | mit S-2222 (0,1 mmol/l) |
| Chymotrypsin (0,2 mg/l) | mit S-2586 (0,1 mmol/l) |
| Plasmin (0,04 CU/ml) | mit S-2251 (0,1 mmol/l) |
| Faktor Xa (0,2 nkat/ml) | mit S-2765 (0,1 mmol/l) |
| aktives Protein C | mit S-2366 (0,2 mmol/l) |

(jeweils Endkonzentration im Test) untersucht. Die Ergebnisse dieser Tests ermöglichen eine Aussage über die Selektivität der Wirkung von Prüfsubstanzen.

### 3. Thrombininduzierte Thrombozytenaggregation in vitro

Frisches humanes Citratblut (9 Teile Blut + 1 Teil Natriumcitrat 0,11 mol/l) wird zur Gewinnung von plättchenreichem Plasma (PRP) und plättchenarmem Plasma (PPP) 16 min bei 250 xg bzw. 20 min bei 3670 xg zentrifugiert.

Zur Bestimmung der Thrombozytenaggregation werden 445 »l PRP mit 5 »l Lösungsmittel (Kontrolle) bzw. Prüfsubstanz versetzt und 5 min bei Raumtemperatur inkubiert. Danach wird der Ansatz 3 min bei 37°C in einem Aggregometer (ELVI 840) inkubiert und 4 min bei 1000 RPM gerührt. Die Aggregation wird durch Zugabe von 50 »l Thrombinlösung (Endkonzentration im Ansatz: 0,15 IU/ml) gestartet. Die Thrombozytenaggregation wird über die Änderung der gemessenen Transmission pro Zeiteinheit in der Probe bestimmt (Slope-Methode).

Als Maß der relativen Wirksamkeit wird als IC₅₀ die Konzentration einer Prüfsubstanz in mol/l berechnet, die die Thrombozytenaggregation um 50 % hemmt.

### 4. Antithrombotische Wirkung am arteriovenösen Shunt an der Ratte

In diesem Experiment dient eine Glaskapillare in einem arteriovenösen Shunt als künstliche thrombogene Oberfläche und löst eine Thrombose aus.

Die narkotisierte (Urethan 25 %, 2 x 8 mg/kp i.p.) Ratte wird in Rückenlage auf einer temperierten (37°C) Wärmebank fixiert. In die freipräparierte rechte A. carotis und V. jugularis werden kurze Polyethylen-Katheter (Portex, PE 50) implantiert, mit physiologischer NaCl-Lösung gefüllt und durch Klemmen verschlossen. Die freien Enden der Katheder werden durch eine 20 mm lange Glaskapillare (Innendurchmesser 1,0 mm) verbunden, die als thrombogene Oberfläche wirkt.

Die Applikation der Prüfsubstanz kann i.v., s.c., p.o. oder als Infusion erfolgen. Nach der gewünschten Inkubationszeit (5, 60 oder 360 min) mit der Prüfsubstanz oder Lösungsmittel (Kontrolle) wird der Shunt durch Entfernen der Klemmen geöffnet. Der Blutstrom durch den Shunt führt zu einem schnellen Anstieg der Shunt-Temperatur, die an der Mitte der Glaskapillare gemessen wird. Die Erhöhung von Raumtemperatur auf Körpertemperatur ist ein Indikator für die Durchgängigkeit des Shunts. Die Temperatur wird bis zum Verschluß des Shunts längstens jedoch 30 min kontinuierlich aufgezeichnet.

Bei Öffnung des Shunts und am Ende des Experiments werden zusätzlich Blutproben zur Bestimmung der anti-FIIa-Aktivität im Plasma entnommen.

Die Versuchsauswertung erfolgt quantitativ. Mit den Werten von log-Dosis und der Zeit (als Differenz der Verschlußzeiten von behandelter Gruppe und Kontrollgruppe) wird eine lineare Regression berechnet. Für den Vergleich der Wirksamkeit unterschiedlicher Testsubstanzen wird aus der Gleichung der Regressionsgeraden der Wert für die ED15min (die Dosis, die bezogen auf die Kontrollgruppe zu einer Verlängerung der Verschlußzeit um 15 min führt) berechnet.

Die neuen Verbindungen sind einfach und in hoher Reinheit herzustellen, chemisch stabil, zeigen kein immunogenes Potential und eignen sich zur Behandlung und Prophylaxe thromboembolischer Erkrankungen wie Myokardinfarkt, peripherer arterieller Verschlußkrankheiten, tiefer Venenthrombosen, Lungenembolie sowie zur Verhinderung der Reocclusion nach Wiedereröffnung arterieller und venöser Gefäße. Sie eignen sich ferner zur Beschichtung künstlicher Oberflächen, die mit Blut oder Plasma in Kontakt stehen (z.B. Dialysemembranen).

Die neuen Verbindungen weisen gegenüber bereits beschriebenen, ebenfalls vom Hirudin abgeleiteten Peptiden, wie MD 28050 (Thrombosis and Haemostasis 63 (1990) 208-214; EP 0 372 503), Hirulog-1 (Biochemistry 29 (1990) 7095-7101; WO 91/02750) und P79 (FEBS Letters 282 (1991) 47-52), eine bessere Wirksamkeit auf. Durch den Einsatz der in der Formel II beschriebenen unnatürlichen Aminosäure mit dem Substituenten W = SO₃H sind beträchtlich wirksamere Peptide synthetisiert worden, als durch den Einsatz der unnatürlichen Aminosäure 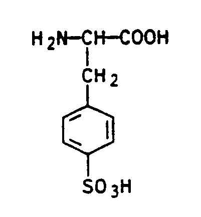
in Peptiden sonst analoger Sequenz. Diese Aminosäure war in anderen von Hirudin abgeleiteten Peptiden bereits eingesetzt worden (EP 0 443 598). Die Wirkstärke dieser neuen Verbindungen liegt in der Größenordnung der Wirkstärke des rekombinanten Hirudins.

### Beispiele

### 1. Synthese der Aminosäuren der Formel II

Die unnatürlichen Aminosäuren der Formel II wurden nach einer in der Literatur beschriebenen Synthese (J. Org. Chem. 53 (1988) 3621-3624; EP 0 354 108) hergestellt.

### 2. Synthese der Peptide der Formel I

### 2.1 Allgemeine Arbeitsvorschrift

Die Synthese der Peptide der Formel I erfolgte mit Hilfe der Standardmethoden der Festphasenpeptidsynthese an einem halbautomatischen Peptidsynthesizer Modell SP650 der Firma LABORTEC. Mengenverhältnisse und Volumina der eingesetzten Reagenzien wurden, soweit nicht anders angegeben, dem Benutzer-Manual entsprechend verwendet. Jeder Kupplungs- und Abspaltungsschritt wurde auf Vollständigkeit überprüft und gegebenenfalls wiederholt.

### 2.1.1 Typischer Synthesecyclus für die Boc-Schutzgruppentechnik:

| | |
|---|---|
| 1. Trifluoroessigsäure und Anisol in DCM | 1 x 5 min |
| (480:20:500) | |
| 2. wie 1. | 1 x 15 min |
| 3. Waschen mit DCM | 3 x 5 min |
| 4. Diisopropylamin in DCM (100:900) | 3 x 3 min |
| 5. Waschen mit DCM | 3 x 5 min |
| 6. Kupplung: 1,5 eq Aminosäure, 1,5 eq 0,5 M TBTU in DMF, 1,5 eq 0,5 M Diisopropylethylamin in DCM | 1 x 3 min |
| 7. Waschen mit DCM | 3 x 5 min |
| 8. bei unvollständigem Umsatz Wiederholung der Kupplung (d.h. zurück zu 6.) oder Capping. | |
| 9. zurück zu 1. | |

### 2.1.2 Typischer Synthesecyclus für die Fmoc-Schutzgruppentechnik

| | |
|---|---|
| 1. Piperidin in DMF (200:800) | 1 x 5 min |
| 2. wie 1. | 1 x 15 min |
| 3. Waschen mit DMF | 2 x 5 min |
| 4. Waschen mit DCM | 2 x 5 min |
| 5. Kupplung wie unter 2.1.1. beschrieben | 1 x 10 min |
| 6. Waschen mit DCM | 2 x 5 min |
| 7. Waschen mit DMF | 2 x 5 min |
| 8. bei unvollständigem Umsatz Wiederholung der Kupplung (d.h. zurück zu 5.) oder Capping | |
| 9. zurück zu 1. | |

### 2.1.3 Aufarbeitung der nach 2.1.1. erhaltenen Peptidharze

Das nach 2.1.1 erhaltene Peptidharz wurde im Vakuum getrocknet und in ein Reaktionsgefäß einer TEFLON®-HF-Apparatur (Fa. PENINSULA) transferiert. Nach Zugabe eines Scavengers, vorzugsweise Anisol (1 ml/g Harz) wurde unter Kühlung mit flüssigem N₂ Fluorwasserstoff einkondensiert (10 ml/g Harz). Man ließ die Mischung sich auf 0°C erwärmen und rührte 45 min bei dieser Temperatur. Anschließend wurde der Fluorwasserstoff im Vakuum abgezogen und der Rückstand mit Essigester gewaschen, um restlichen Scavenger zu entfernen. Das Peptid wurde mit 30 %iger Essigsäure extrahiert, filtriert und das Filtrat lyophilisiert.

### 2.1.4 Aufarbeitung der nach 2.1.2 erhaltenen Peptidharze

Das gemäß 2.1.2. erhaltene Peptidharz wurde im Vakuum getrocknet und anschließend in Abhängigkeit von der Aminosäurezusammensetzung einer der folgenden Spaltungsprozeduren unterworfen (Wade, Tregear, Howard Florey Fmoc-Workshop Manual, Melbourne 1985).

| Das Peptid enthielt | | | Spaltbedingungen | | |
|---|---|---|---|---|---|
| Arg(Mtr) | Met | Trp | TFA | Scavenger | Reaktionszeit |
| nein | nein | nein | 95 % | 5 % H₂ | 1,5 h |
| ja | nein | nein | 95 % | 5 % Thioanisol | 3 h |
| nein | ja | nein | 95 % | 5 % Ethylmethylsulfid | 1,5 h |
| nein | nein | ja | 95 % | 5 % Ethandithiol/Anisol (1:3) | 1,5 h |
| nein | ja | ja | 95 % | 5 % Ethandithiol/Anisol/Ethylmethylsulfid (1:3:1) | 1,5 h |
| ja | ja | ja | 93 % | 7 % Ethandithiol/Anisol/Ethylmethylsulfid (1:3:3) | 3 h |

Die Suspension des Peptidharzes in der geeigneten TFA-Mischung wurde bei Raumtemperatur für die angegebene Zeit gerührt, danach wurde das Harz abfiltriert und mit TFA sowie DCM gewaschen. Das Filtrat und die Waschlösungen wurden weitgehend eingeengt und das Peptid durch Zugabe von Diethylether ausgefällt. Nach Abkühlung im Eisbad wurde der Niederschlag abfiltriert, in 30 % Essigsäure aufgenommen und lyophilisiert.

### 2.1.5 Reinigung und Charakterisierung der Peptide

Die Qualität der Rohpeptide wie auch der gereinigten Peptide wurde mit Hilfe der HPLC beurteilt; die Reinigung wurde mit MPLC oder einer Kombination aus Gelchromatographie und MPLC vorgenommen.

HPLC:
Gerät: HP 1090 Liquid Chromatograph
Stationäre Phase: 100 x 2,1 mm VYDAC C18 (TPB 218); 5 »m; 300 Å
Mobile Phase: (A) 0,1 % TFA in H₂O; (B) 0,1 % TFA in CH₃CN;
Fluß: 0,2 ml/min; Temp. 40°C
Gradienten:
(a): ab 0 % (B) mit 0,5 % min⁻¹
(b): ab 5 % (B) mit 1,0 % min⁻¹
(c): ab 35 % (B) mit 1,0 % min⁻¹
Probenaufgabe: 3 »l; etwa 0,03 %ige Lösung im Eluenten Detektion: 205 nm
MPLC:
Gerät:
Labomatic-Gradienten-Mitteldruckchromatographie-Station
Stationäre Phase: 26 x 450 mm bis 37 x 450 mm Eurosil Bioselect 100/30 C 18; 20 - 45 »m; 100 Å
Mobile Phase: (A) 0,1 % TFA in H₂O; (B) CH₃CN
Gradient: Stufengradient mit 0,25 % min⁻¹ während der Elutionsphase
Detektion: 205 bis 220 nm
Gelchromatographie:
Stationäre Phasen: SEPHADEX® G-10 bzw. -LH20
Mobile Phasen: 10 % AcOH bzw. MeOH
Detektion: UV- und RI-Detektoren in Serie geschaltet.

Zur Charakterisierung der gereinigten Peptide wurden Aminosäureanalyse (AAA), Fast-Atom-Bombardment-Massenspektrometrie (FAB-MS) und gelegentlich Sequencing (SQ) oder NMR-Spektroskopie (NMR) herangezogen.

### 2.2 Spezielle Arbeitsvorschriften

### 2.2.1 [p-Sulfo-Phe⁶³]-Nα-acetyl-hirudin₅₆₋₆₅

1,00 g (0,47 meq) Boc-Gln-Pam-Harz wurden gemäß 2.1.1 umgesetzt mit:

| | |
|---|---|
| Boc-Leu-OH | Boc-Ile-OH |
| Boc-(p-Sulfo)Phe-OH | Boc-Glu(OChx)-OH |
| Boc-Glu(OChx)-OH | Boc-Glu(OChx)-OH |
| Boc-Glu(OChx)-OH | Boc-Phe-OH |
| Boc-Pro-OH | |

Nach der N-Acetylierung mit Acetanhydrid wurde gemäß 2.1.3 aufgearbeitet:
Rohausbeute: 305 mg Lyophilisat
Das Rohpeptid wurde gemäß 2.1.5 durch MPLC gereinigt.

HPLC-Retentionszeit (Gradient b): 19,5 min; AAA: stimmt; FAB-MS: 1402

### 2.2.2 [D-p-Sulfomethyl-Phe⁶³]-Nα-Acetyl-Hirudin₅₆₋₆₅ und [L-p-Sulfomethyl-Phe⁶³]-Nα-Acetyl-Hirudin₅₆₋₆₅

2,0 g (1,54 meq) Boc-Gln-Pam-Harz wurde gemäß 2.1.1 umgesetzt mit:

| | |
|---|---|
| Boc-Leu-OH | Boc-Ile-OH |
| Boc-D,L-(p-Sulfomethyl)Phe-OH | Boc-Glu(OChx)-OH |
| Boc-Glu(OChx)-OH | Boc-Glu(OChx)-OH |
| Boc-Glu(OChx)-OH | Boc-Phe-OH |
| Boc-Pro-OH | |

Nach der N-Acetylierung mit Acetanhydrid wurde gemäß 2.1.3 aufgearbeitet:
Rohausbeute: 304 mg Lyophilisat
Das Rohpeptid wurde gemäß 2.1.5 durch MPLC gereinigt, wobei sich die beiden Diastereomeren trennen ließen.

HPLC-Retentionszeiten (Gradient b): 18,5 und 19,5 min; AAA: stimmt; FAB-MS: 1416 und 1416
Analog ließen sich [D-p-Phosphonomethyl-Phe⁶³]-Nα-acetyl-hirudin₅₆₋₆₅ und [L-p-Phosphonomethyl-Phe⁶³]-Nα-acetyl-hirudin₅₆₋₆₅ [HPLC-Retentionszeiten (Gradient b): 19,5 und 21,0 min; AAA: stimmt; FAB-MS: 1418 und 1418] darstellen.

### 2.2.3 [D-p-Sulfomethyl-Phe⁶³]-N^{α}-succinyl-hirudin₅₆₋₆₅ und [L-p-Sulfomethyl-Phe⁶³]-N^{α}-succinyl-hirudin₅₆₋₆₅

2,0 g (1,54 meq) Boc-Gln-Pam-Harz wurden gemäß 2.1.1 umgesetzt mit:

| | |
|---|---|
| Boc-Leu-OH | Boc-Ile-OH |
| Boc-D,L-(p-Sulfomethyl)Phe-OH | Boc-Glu(OChx)-OH |
| Boc-Glu(OChx)-OH | Boc-Glu(OChx)-OH |
| Boc-Glu(OChx)-OH | Boc-Phe-OH |
| Boc-Pro-OH | |

Nach der N-Succinylierung mit Bernsteinsäureanhydrid wurde gemäß 2.1.3 aufgearbeitet:
Rohausbeute: 364 mg Lyophilisat
Das Rohpeptid wurde gemäß 2.1.5 durch MPLC gereinigt.

HPLC-Retentionszeit (Gradient b): 18,5 min und 20,0 min; AAA: stimmt; FAB-MS: 1474 und 1474
Analog ließen sich darstellen:
[p-Sulfo-Phe⁶³]-N^{α}-succinyl-hirudin₅₆₋₆₅
HPLC-Retentionszeit (Gradient b): 23,0 min; AAA: stimmt; FAB-MS: 1460
[Hyp⁶⁰,p-Sulfo-Phe⁶³]-N^{α}-succinyl-hirudin₅₆₋₆₅
HPLC-Retentionszeit (Gradient b): 20,0 min; AAA: stimmt; FAB-MS: 1476
[Hyp⁶⁰,p-Sulfo-Phe⁶³,Cha⁶⁴]-N^{α}-succinyl-hirudin₅₆₋₆₅
HPLC-Retentionszeit (Gradient b): 25,0 min; AAA: stimmt; FAB-MS: 1516
[Tyr⁵⁶,D-p-Sulfomethyl-Phe⁶³,Cha⁶⁴]-N^{α}-succinyl-hirudin₅₆₋₆₅ und
[Tyr⁵⁶,L-p-Sulfomethyl-Phe⁶³,Cha⁶⁴]-N^{α}-succinyl-hirudin₅₆₋₆₅
HPLC-Retentionszeit (Gradient b): 23,0 min und 26,5 min; AAA: stimmt; FAB-MS: 1530 und 1530
[Tyr⁵⁶,Pro⁵⁸,D-p-Sulfomethyl-Phe⁶³,Cha⁶⁴]-N^{α}-succinyl-hirudin₅₆₋₆₅ und
[Tyr⁵⁶,Pro⁵⁸,L-p-Sulfomethyl-Phe⁶³,Cha⁶⁴]-N^{α}-succinyl-hirudin₅₆₋₆₅
HPLC-Retentionszeit (Gradient b): 18,0 min und 19,5 min; AAA: stimmt; FAB-MS: 1498 und 1498
[Tyr⁵⁶,Pro⁵⁸,Hyp⁶⁰,D-p-Sulfomethyl-Phe⁶³,Cha⁶⁴]-N^{α}-succinyl-hirudin₅₆₋₆₅ und
[Tyr⁵⁶,Pro⁵⁸,Hyp⁶⁰,L-p-Sulfomethyl-Phe⁶³,Cha⁶⁴]-N^{α}-succinyl-hirudin₅₆₋₆₅
HPLC-Retentionszeit (Gradient b): 19,0 min und 20,0 min; AAA: stimmt; FAB-MS: 1514 und 1514
[Tyr⁵⁶,Pro⁵⁸,Hyp⁶⁰,D-p-Sulfomethyl-Phe⁶³,Cha⁶⁴,Glu⁶⁵]-N^{α}-succinyl-hirudin₅₆₋₆₅ und
[Tyr⁵⁶,Pro⁵⁸,Hyp⁶⁰,L-p-Sulfomethyl-Phe⁶³,Cha⁶⁴,Glu⁶⁵]-N^{α}-succinyl-hirudin₅₆₋₆₅
HPLC-Retentionszeit (Gradient b): 20,0 min und 23,0 min; AAA: stimmt; FAB-MS: 1515 und 1515
Bei Einsatz von Racematen der Aminosäuren der Formel II in die Peptidsynthese wurden die resultierenden diastereomeren Peptidgemische mit Hilfe der MPLC getrennt. In der Regel zeigte dabei das diastereomere Peptid mit der größeren HPLC-Retentionszeit eine um zwei Größenordnungen bessere Wirkstärke als das Diastereomere mit der kleineren HPLC-Retentionszeit. Eine Zuordnung bezüglich der jeweils enthaltenden Enantiomeren der unnatürlichen Aminosäure der Formel II konnte bisher noch nicht erfolgen.

### 2.3 Weitere Beispiele für Peptide der Formel I

Wie unter Punkt 2.2 beschrieben, können folgende Peptide der Formel I synthetisiert werden:
[D-p-Sulfomethyl-Phe⁶³,Cha⁶⁴]-N^{α}-succinyl-hirudin₅₆₋₆₅
[L-p-Sulfomethyl-Phe⁶³,Cha⁶⁴]-N^{α}-succinyl-hirudin₅₆₋₆₅
[Hyp⁶⁰,D-p-Sulfomethyl-Phe⁶³]-N^{α}-succinyl-hirudin₅₆₋₆₅
[Hyp⁶⁰,L-p-Sulfomethyl-Phe⁶³]-N^{α}-succinyl-hirudin₅₆₋₆₅
[Hyp⁶⁰,D-p-Sulfomethyl-Phe⁶³,Cha⁶⁴]-N^{α}-succinyl-hirudin₅₆₋₆₅
[Hyp⁶⁰,L-p-Sulfomethyl-Phe⁶³,Cha⁶⁴]-N^{α}-succinyl-hirudin₅₆₋₆₅
[Tyr⁵⁶,Hyp⁶⁰,D-p-Sulfomethyl-Phe⁶³,Cha⁶⁴]-N^{α}-succinyl-hirudin₅₆₋₆₅
[Tyr⁵⁶,Hyp⁶⁰,L-p-Sulfomethyl-Phe⁶³,Cha⁶⁴]-N^{α}-succinyl-hirudin₅₆₋₆₅
[Tyr⁵⁶,Hyp⁵⁸,Hyp⁶⁰,D-p-Sulfomethyl-Phe⁶³,Cha⁶⁴]-N^{α}-succinyl-hirudin₅₆₋₆₅
[Tyr⁵⁶,Hyp⁵⁸,Hyp⁶⁰,L-p-Sulfomethyl-Phe⁶³,Cha⁶⁴]-N^{α}-succinyl-hirudin₅₆₋₆₅
[Tyr⁵⁶,Pro⁵⁸,Val⁵⁹,Hyp⁶⁰,D-p-Sulfomethyl-Phe⁶³,Cha⁶⁴]-N^{α}-succinyl-hirudin₅₆₋₆₅
[Tyr⁵⁶,Pro⁵⁸,Val⁵⁹,Hyp⁶⁰,L-p-Sulfomethyl-Phe⁶³,Cha⁶⁴]-N^{α}-succinyl-hirudin₅₆₋₆₅
[Tyr⁵⁶,Pro⁵⁸,Cha⁵⁹,Hyp⁶⁰,D-p-Sulfomethyl-Phe⁶³,Ile⁶⁴]-N^{α}-succinyl-hirudin₅₆₋₆₅
[Tyr⁵⁶,Pro⁵⁸,Cha⁵⁹,Hyp⁶⁰,L-p-Sulfomethyl-Phe⁶³,Ile⁶⁴]-N^{α}-succinyl-hirudin₅₆₋₆₅
[Tyr⁵⁶,Pro⁵⁸,Hyp⁶⁰,D-p-Sulfomethyl-Phe⁶³,Cha⁶⁴]-N^{α}-succinyl-hirudin₅₆₋₆₄
[Tyr⁵⁶,Pro⁵⁸,Hyp⁶⁰,L-p-Sulfomethyl-Phe⁶³,Cha⁶⁴]-N^{α}-succinyl-hirudin₅₆₋₆₄
[Tyr⁵⁶,Pro⁵⁸,Hyp⁶⁰,D-p-Sulfomethyl-Phe⁶³,Cha⁶⁴,D-Gln⁶⁵]-N^{α}-succinyl-hirudin₅₆₋₆₅
[Tyr⁵⁶,Pro⁵⁸,Hyp⁶⁰,L-p-Sulfomethyl-Phe⁶³,Cha⁶⁴,D-Gln⁶⁵]-N^{α}-succinyl-hirudin₅₆₋₆₅
[Tyr⁵⁶,Pro⁵⁸,Hyp⁶⁰,D-p-Sulfomethyl-Phe⁶³,Cha⁶⁴,D-Glu⁶⁵]-N^{α}-succinyl-hirudin₅₆₋₆₅
[Tyr⁵⁶,Pro⁵⁸,Hyp⁶⁰,L-p-Sulfomethyl-Phe⁶³,Cha⁶⁴,D-Glu⁶⁵]-N^{α}-succinyl-hirudin₅₆₋₆₅
[Tyr⁵⁶,Hyp⁵⁸,Val⁵⁹,Hyp⁶⁰,D-p-Sulfomethyl-Phe⁶³,Cha⁶⁴]-N^{α}-succinyl-hirudin₅₆₋₆₅
[Tyr⁵⁶,Hyp⁵⁸,Val⁵⁹,Hyp⁶⁰,L-p-Sulfomethyl-Phe⁶³,Cha⁶⁴]-N^{α}-succinyl-hirudin₅₆₋₆₅
[Tyr⁵⁶,Hyp⁵⁸,Cha⁵⁹,Hyp⁶⁰,D-p-Sulfomethyl-Phe⁶³,Ile⁶⁴]-N^{α}-succinyl-hirudin₅₆₋₆₅
[Tyr⁵⁶,Hyp⁵⁸,Cha⁵⁹,Hyp⁶⁰,L-p-Sulfomethyl-Phe⁶³,Ile⁶⁴]-N^{α}-succinyl-hirudin₅₆₋₆₅
[D-p-Phosphonomethyl-Phe⁶³]-N^{α}-succinyl-hirudin₅₆₋₆₅
[L-p-Phosphonomethyl-Phe⁶³]-N^{α}-succinyl-hirudin₅₆₋₆₅
[D-p-Phosphonomethyl-Phe⁶³,Cha⁶⁴]-N^{α}-succinyl-hirudin₅₆₋₆₅
[L-p-Phosphonomethyl-Phe⁶³,Cha⁶⁴]-N^{α}-succinyl-hirudin₅₆₋₆₅
[Tyr⁵⁶,D-p-Phosphonomethyl-Phe⁶³,Cha⁶⁴]-N^{α}-succinyl-hirudin₅₆₋₆₅
[Tyr⁵⁶,L-p-Phosphonomethyl-Phe⁶³,Cha⁶⁴]-N^{α}-succinyl-hirudin₅₆₋₆₅
[Tyr⁵⁶,Pro⁵⁸,D-p-Phosphonomethyl-Phe⁶³,Cha⁶⁴]-N^{α}-succinyl-hirudin₅₆₋₆₅
[Tyr⁵⁶,Pro⁵⁸,L-p-Phosphonomethyl-Phe⁶³,Cha⁶⁴]-N^{α}-succinyl-hirudin₅₆₋₆₅
[Hyp⁶⁰,D-p-Phosphonomethyl-Phe⁶³]-N^{α}-succinyl-hirudin₅₆₋₆₅
[Hyp⁶⁰,L-p-Phosphonomethyl-Phe⁶³]-N^{α}-succinyl-hirudin₅₆₋₆₅
[Hyp⁶⁰,D-p-Phosphonomethyl-Phe⁶³,Cha⁶⁴]-N^{α}-succinyl-hirudin₅₆₋₆₅
[Hyp⁶⁰,L-p-Phosphonomethyl-Phe⁶³,Cha⁶⁴]-N^{α}-succinyl-hirudin₅₆₋₆₅
[Tyr⁵⁶,Hyp⁶⁰,D-p-Phosphonomethyl-Phe⁶³,Cha⁶⁴]-N^{α}-succinyl-hirudin₅₆₋₆₅
[Tyr⁵⁶,Hyp⁶⁰,L-p-Phosphonomethyl-Phe⁶³,Cha⁶⁴]-N^{α}-succinyl-hirudin₅₆₋₆₅
[Tyr⁵⁶,Pro⁵⁸,Hyp⁶⁰,D-p-Phosphonomethyl-Phe⁶³,Cha⁶⁴]-N^{α}-succinyl-hirudin₅₆₋₆₅
[Tyr⁵⁶,Pro⁵⁸,Hyp⁶⁰,L-p-Phosphonomethyl-Phe⁶³,Cha⁶⁴]-N^{α}-succinyl-hirudin₅₆₋₆₅
[Tyr⁵⁶,Hyp⁵⁸,Hyp⁶⁰,D-p-Phosphonomethyl-Phe⁶³,Cha⁶⁴]-N^{α}-succinyl-hirudin₅₆₋₆₅
[Tyr⁵⁶,Hyp⁵⁸,Hyp⁶⁰,L-p-Phosphonomethyl-Phe⁶³,Cha⁶⁴]-N^{α}-succinyl-hirudin₅₆₋₆₅
[Tyr⁵⁶,Pro⁵⁸,Val⁵⁹,Hyp⁶⁰,D-p-Phosphonomethyl-Phe⁶³,Cha⁶⁴]-N^{α}-succinyl-hirudin₅₆₋₆₅
[Tyr⁵⁶,Pro⁵⁸,Val⁵⁹,Hyp⁶⁰,L-p-Phosphonomethyl-Phe⁶³,Cha⁶⁴]-N^{α}-succinyl-hirudin₅₆₋₆₅
[Tyr⁵⁶,Pro⁵⁸,Cha⁵⁹,Hyp⁶⁰,D-p-Phosphonomethyl-Phe⁶³,Ile⁶⁴]-N^{α}-succinyl-hirudin₅₆₋₆₅
[Tyr⁵⁶,Pro⁵⁸,Cha⁵⁹,Hyp⁶⁰,L-p-Phosphonomethyl-Phe⁶³,Ile⁶⁴]-N^{α}-succinyl-hirudin₅₆₋₆₅
[Tyr⁵⁶,Pro⁵⁸,Hyp⁶⁰,D-p-Phosphonomethyl-Phe⁶³,Cha⁶⁴]-N^{α}-succinyl-hirudin₅₆₋₆₄
[Tyr⁵⁶,Pro⁵⁸,Hyp⁶⁰,L-p-Phosphonomethyl-Phe⁶³,Cha⁶⁴]-N^{α}-succinyl-hirudin₅₆₋₆₄
[Tyr⁵⁶,Pro⁵⁸,Hyp⁶⁰,D-p-Phosphonomethyl-Phe⁶³,Cha⁶⁴, D-Gln⁶⁵]-N^{α}-succinyl-hirudin₅₆₋₆₅
[Tyr⁵⁶,Pro⁵⁸,Hyp⁶⁰,L-p-Phosphonomethyl-Phe⁶³,Cha⁶⁴, D-Gln⁶⁵]-N^{α}-succinyl-hirudin₅₆₋₆₅
[Tyr⁵⁶,Pro⁵⁸,Hyp⁶⁰,D-p-Phosphonomethyl-Phe⁶³,Cha⁶⁴, Glu⁶⁵]-N^{α}-succinyl-hirudin₅₆₋₆₅
[Tyr⁵⁶,Pro⁵⁸,Hyp⁶⁰,L-p-Phosphonomethyl-Phe⁶³,Cha⁶⁴, Glu⁶⁵]-N^{α}-succinyl-hirudin₅₆₋₆₅
[Tyr⁵⁶,Pro⁵⁸,Hyp⁶⁰,D-p-Phosphonomethyl-Phe⁶³,Cha⁶⁴, D-Glu⁶⁵]-N^{α}-succinyl-hirudin₅₆₋₆₅
[Tyr⁵⁶,Pro⁵⁸,Hyp⁶⁰,L-p-Phosphonomethyl-Phe⁶³,Cha⁶⁴, D-Glu⁶⁵]-N^{α}-succinyl-hirudin₅₆₋₆₅
[Tyr⁵⁶,Hyp⁵⁸,Val⁵⁹,Hyp⁶⁰,D-p-Phosphonomethyl-Phe⁶³,Cha⁶⁴]-N^{α}-succinyl-hirudin₅₆₋₆₅
[Tyr⁵⁶,Hyp⁵⁸,Val⁵⁹,Hyp⁶⁰,L-p-Phosphonomethyl-Phe⁶³,Cha⁶⁴]-N^{α}-succinyl-hirudin₅₆₋₆₅
[Tyr⁵⁶,Hyp⁵⁸,Cha⁵⁹,Hyp⁶⁰,D-p-Phosphonomethyl-Phe⁶³,Ile⁶⁴]-N^{α}-succinyl-hirudin₅₆₋₆₅
[Tyr⁵⁶,Hyp⁵⁸,Cha⁵⁹,Hyp⁶⁰,L-p-Phosphonomethyl-Phe⁶³,Ile⁶⁴]-N^{α}-succinyl-hirudin₅₆₋₆₅

## Patentansprüche

1. Peptide der Formel I 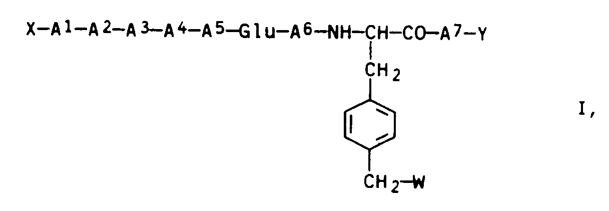 worin
X H, Acetyl, Succinyl, Maleoyl, Phthaloyl oder Fumaryl,
A¹ Phe, Tyr oder Cha,
A² Glu oder D-Glu,
A³ Glu, Pro oder Hyp,
A⁴ Ile, Leu, Val oder Cha,
A⁵ Pro oder Hyp,
A⁶ Glu oder D-Glu,
A⁷ Leu, D-Leu, Ile, D-Ile, Cha oder D-Cha,
Y OH, Gln-OH, Gln-NH₂, D-Gln-OH, D-Gln-NH₂, Glu-OH, Glu-NH₂, D-Glu-OH oder D-Glu-NH₂ und
W für einen der Reste SO₃H oder PO₃H₂ steht,
sowie deren Salze mit physiologisch verträglichen Basen oder Säuren.

2. Verfahren zur Herstellung der Peptide der Formel I gemäß Anspruch 1, dadurch gekennzeichnet, daß man diese nach in der Peptidchemie bekannten Methoden herstellt, ausgegangen von den einzelnen Aminosäuren die aneinander gekuppelt werden.

3. Peptide der Formel I gemäß Anspruch 1 zur Verwendung bei der Bekämpfung von Krankheiten.

## Claims

1. A peptide of the formula I 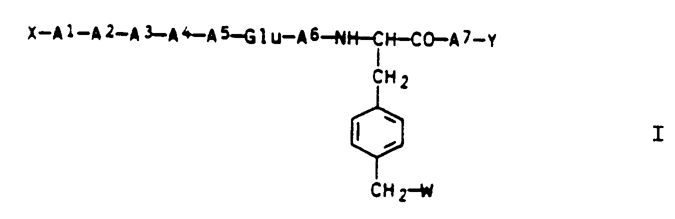 where
X is H, acetyl, succinyl, maleoyl, phthaloyl or fumaroyl,
A¹ is Phe, Tyr or Cha,
A² is Glu or D-Glu,
A³ is Glu, Pro or Hyp,
A⁴ is Ile, Leu, Val or Cha,
A⁵ is Pro or Hyp,
A⁶ is Glu or D-Glu,
A⁷ is Leu, D-Leu, Ile, D-Ile, Cha or D-Cha,
Y is OH, Gln-OH, Gln-NH₂, D-Gln-OH, D-Gln-NH₂, Glu-OH, Glu-NH₂, D-Glu-OH or D-Glu-NH₂ and
W is SO₃H or PO₃H₂,
and the salts thereof with physiologically tolerated bases or acids.

2. A process for preparing a peptide of the formula I as claimed in claim 1, wherein the preparation is carried out by methods known in peptide chemistry, starting from the individual amino acids and coupling them to one another.

3. A peptide of the formula I as claimed in claim 1 for use in controlling diseases.

## Revendications

1. Peptides de la formule I 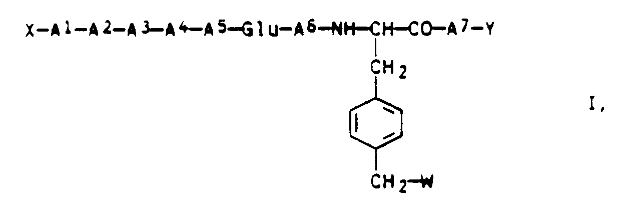 dans laquelle représentent
X H, acétyle, succinyle, maléoyle, phtaloyle ou fumaryle,
A¹ Phe, Tyr ou Cha,
A² Glu ou D-Glu,
A³ Glu, Pro ou Hyp,
A⁴ Ile, Leu, Val ou Cha,
A⁵ Pro ou Hyp,
A⁶ Glu ou D-Glu,
A⁷ Leu, D-Leu, Ile, D-Ile, Cha ou D-Cha,
Y OH, Gln-OH, Gln-NH₂, D-Gln-OH, D-Gln-NH₂, Glu-OH, Glu-NH₂, D-Glu-OH ou D-Glu-NH₂ et
W représente l'un des radicaux SO₃H ou PO₃H₂,
ainsi que leurs sels avec des acides ou des bases physiologiquement compatibles.

2. Procédé de préparation des peptides de la formule I suivant la revendication 1, caractérisé en ce qu'on prépare ceux-ci selon des méthodes bien connues de la chimie des peptides, en partant des aminoacides individuels couplés les uns aux autres.

3. Peptides de la formule I suivant la revendication 1, destinés à l'emploi pour la lutte contre des maladies.
